# EUROPÄISCHE PATENTSCHRIFT

(11) **EP 1 857 081 B1**
(45) Veröffentlichungstag und Bekanntmachung des Hinweises auf die Patenterteilung: **29.06.2016**
(21) Anmeldenummer: 07005857.3
(22) Anmeldetag: 22.03.2007
(51) Int. Cl.: A61F 2/78, A61F 2/72

(54) **Protheseninnenschaftsystem**
Prosthesis inner shaft system
Système de tige intérieure de prothèse

(30) Priorität: 09.05.2006 DE 202006007460 U
(43) Veröffentlichungstag der Anmeldung: 21.11.2007
(73) Patentinhaber: Otto Bock Healthcare Products GmbH, 1070 Wien (AT)
(72) Erfinder: Klein, Christian, 1070 Wien (AT)
(74) Vertreter: Stornebel, Kai

(56) Entgegenhaltungen:
- EP-A1- 0 765 646
- WO-A-02/085264
- WO-A-03/086245
- WO-A-2005/039444
- GB-A- 1 191 301
- GB-A- 1 191 633
- US-A- 2 696 011
- US-A- 5 413 611
- US-A- 5 443 525
- US-A- 5 980 577
- US-B1- 6 673 117

## Beschreibung

Die Erfindung betrifft ein Protheseninnenschaftsystem mit einem Innenschaft, der in einem Prothesenaußenschaft mit diesem verbindbar gelagert ist.

Prothetische Elemente, wie Prothesenkniegelenke, künstliche Unterschenkel oder Kunsthände werden an Prothesenschäften befestigt, die an dem entsprechenden Stumpf des Prothesennutzers festgelegt werden. Je nach gewünschtem Funktionsumfang wird dabei zwischen rein kosmetischen Prothesen, mechanisch betätigten Prothesen oder bioelektrisch gesteuerten Prothesen unterschieden. Die jeweils zu ersetzenden Extremitäten oder Funktionselemente oder Antriebe sind dabei an dem sogenannten Außenschaft gelagert, der an einem unmittelbar an dem Stumpf anliegenden Innenschaft festlegbar ist.

Bei myoelektrisch gesteuerten Prothesen ist die Ableitung eines elektrischen Aktionspotentials von der Stumpfmuskulatur notwendig. Diese Potentiale entstehen bei der Kontraktion eines Muskels und sind auf der Hautoberfläche des Prothesenträgers messbar. Die von der Elektrode abgenommenen Potentiale werden verstärkt und an eine Steuereinheit geleitet, die Aktuatoren aktiviert oder deaktiviert.

Die US 2,696,011 A1 betrifft eine Unterschenkelprothese mit einem Außenschaft und einem darin angeordneten Innenschaft aus mehreren Schichten eines elastischen Materials. Der Innenschaft kann an dem steifen Außenschaft festgelegt werden. Ein Einwegeventil ist im Innenschaft eingeschraubt, um bei jedem Belasten des Prothesenbeins Luft aus dem Zwischenraum zwischen dem Stumpf und der Innenseite des Innenschaftes nach außen zu drücken, damit der Innenschaft an dem Stumpf haften bleibt. Das Ventil ist in einem Bolzen mit einem Außengewinde angeordnet und federbelastet in einer Schließstellung gehalten. Auf dem Außengewinde ist eine Scheibe aufgeschraubt, die das Ventil an dem Innenschaft festlegt,

Die EP 0 765 646 A1 beschreibt eine Unterschenkelprothese mit einem Innenschaft, der eine Luftkammer aufweist. Der Innenschaft ist zum Stumpf hin vollständig geschlossen, die Luftkammer steht über eine Durchgangsbohrung in einem Steckzapfen mit der Umgebung in Verbindung.

Die US 5,443,525 A zeigt einen Silikonliner mit einem fensterartigen Ausschnitt, in dem eine Kontaktmatte befestigt ist, so dass die Kontaktmatte und die innerhalb der Kontaktmatte angeordneten Elektroden relativ zu dem Liner unbeweglich sind. Der fensterartige Ausschnitt wird durch die Matte verschlossen.

Die US 4,413,611 A betrifft eine computergesteuerte Handprothese mit einem Schaft, an dessen Innenseite Elektroden angeordnet sind. Über Muskelkontraktionen wird ein elektrisches Signal erzeugt, um die Prothesenhand zu steuern.

Die US 6,673,117 B1 betrifft ein Prothesenknie mit einem Aktuator, der einen Servoantrieb und Einrichtungen zum Erfassen myoelektrischer Signale an dem Beinstumpf aufweist. Über die myoelektrischen Signale kann der Servomotor angesteuert werden, um ein Steuerelement zu manipulieren.

Die gattungsgemäße US 5,980,577 A betrifft ein Prothesensystem mit einem Außenschaft und einem Liner. In dem Liner sind Öffnungen in der Nähe eines Auslasses aus dem Schaft angeordnet, um Luft aus dem Liner zu entfernen. In dem Außenschaft ist ein Einwegeventil in einem Stopfen eingesetzt.

Die GB 1,191,301 A betrifft eine Protheseneinrichtung mit Elektroden zur Erfassung myoelektrischer Signale zur Steuerung von Bewegungen einer künstlichen Gliedmaße. Ein Aufnahmeteil ist zur Anbringung an dem Körper des Nutzers ausgebildet und weist zwei Öffnungen auf, an denen myoelektrische Signale während einer Muskelkontraktion abgegriffen werden. Elektroden sind verschieblich in den Öffnungen in einem Elektrodenhalter angeordnet, der entfernbar an dem Abstützteil befestigt ist.

Aus dem Stand der Technik ist es bekannt, den Innenschaft mit einer Aussparung zu versehen, in die die Elektrode eingesetzt werden kann. Zwischen der Elektrode und der Aussparung im Innenschaft ist ein gewisses Spaltmaß vorhanden, was zu einer Relativbewegung zwischen Elektrode und Innenschaft führen kann. Durch den Spalt zwischen dem Innenschaft und der Elektrode kann Schweiß in den Zwischenraum zwischen Innenschaft und Außenschaft eindringen, der die elektrischen und elektronischen Komponenten angreifen kann. Ebenfalls kann das Reinigen des Innenschaftes erschwert sein.

Aufgabe der vorliegenden Erfindung ist es, ein verbessertes Protheseninnenschaftsystem bereitzustellen, bei dem diese Nachteile nicht auftreten. Erfindungsgemäß wird diese Aufgabe durch ein Protheseninnenschaftsystem mit den Merkmalen des Anspruchs 1 gelöst. Vorteilhafte Ausgestaltungen und Weiterbildungen der Erfindung sind in den Unteransprüchen beschrieben.

Das erfindungsgemäße Protheseninnenschaftsystem mit einem Innenschaft, der in einem Prothesenaußenschaft mit Aufnahmeeinrichtungen für weitere Prothesenkomponenten verbindbar ausgebildet ist und zumindest eine Ausnehmung zur Durchleitung myoelektrischer Signale einer an dem Innenschaft gelagerten Elektrode aufweist sieht vor, dass die Elektrode in einer die Ausnehmung dichtend abschließenden Halterung befestigt ist. Dadurch ist es möglich, den Innenschaft als einen Saugschaft auszubilden, so dass kein Spalt zwischen dem Innenschaft und der Elektrode bzw. der Elektrodenhalterung vorhanden ist. Luft oder Schweiß kann dann nicht zwischen der Elektrodenhalterung und dem Innenschaft eindringen bzw. hinausdringen. Dadurch sind die restlichen Prothesenkomponenten, zum Beispiel elektronische Komponenten oder der Akkumulator gegen Schweiß geschützt. Bei einer Reinigung oder auch bei der Anwendung von Cremes können keine Stoffe in den Außenschaft gelangen. Durch die luftdichte Montage der Halterung mit der Elektrode an dem Innenschaft bleibt ein Vakuumeffekt des Schaftes erhalten, so dass ein besserer Kontakt zwischen dem Stumpf und der Elektrode sowie ein sicherer Halt des Innenschaftes an dem Stumpf gewährleistet sind. Darüber hinaus wird die Herstellung des Innenschaftes erleichtert, der als laminierter oder tiefgezogener Innenschaft ausgebildet sein kann, indem die Ausnehmung nach dem Herausschlagen des Innenschaftes aus dem Stumpfmodell hineingefräst wird. Die Halterung kann dann separat in die Ausnehmung eingesetzt werden.

Die Halterung besteht dabei bevorzugt aus einem elastischen Material, insbesondere Silikon oder einem anderen hautfreundlichen bzw. hautverträglichen Material. Die Elektrode kann in die Halterung eingespritzt sein, beispielsweise im Rahmen eines Zweikomponentenspritzverfahrens, oder eingeklebt, eingepresst oder formschlüssig darin gehalten werden. Dadurch ist in der Halterung die myoelektrische Elektrode integriert und kann in den Innenschaft eingesetzt werden.

Die Halterung ist bevorzugt mit einer Nut ausgebildet, in die in der montierten Stellung der Innenschaft eingreift und eine formschlüssige und abdichtende Lagerung verwirklicht. Bevorzugt ist die Nut umlaufend in der Halterung ausgebildet, so dass die Halterung innenseitig und außenseitig den Rand der Ausnehmung vollständig überdeckt bzw. übergreift. Somit wird der Innenschaft an der Halterung zwischen zwei Dichtlippen, die eng an dem Innenschaft anliegen, eingeschlossen.

Eine Weiterbildung der Erfindung sieht vor, dass der Innenschaft mit dem Außenschaft, an dem weitere Prothesenpassteile angeordnet sind, mit einem Rohr verbunden ist. Dieses Rohr wird zum Einziehen des Stumpfes in den Innenschaft benötigt. An dem Innenschaft ist bevorzugt ein Einwegeventil befestigt, das die im Innenschaft befindliche Luft herausdrückt, und zwar bei jeder Bewegung des Stumpfes in dem Innenschaft. Das Ventil kann in dem Verbindungsrohr zwischen dem Innenschaft und dem Außenschaft angeordnet sein und verschließt dieses. Durch eine entsprechende Abstimmung des Ventils an das Verbindungsrohr wird zusätzlich verhindert, dass Weichteile in das Verbindungsrohr gezogen werden.

Die Halterung ist bevorzugt federnd oder nachgiebig ausgebildet, um Ungleichmäßigkeiten in der Stumpfform auszugleichen, so dass die Elektrodenkontakte stets gut auf der Haut aufliegen.

Bevorzugt ist die dem Stumpf zugewandte Seite der Halterung abgeflacht, insbesondere kalottenförmig ausgebildet, so dass aufgrund der Form des Spritzteiles mit der integrierten Elektrode keine oder nur geringe Druckstellen auftreten, da sich die Halterung von der Oberfläche der Innenschaftseite kaum abhebt.

Zur besseren Einbringung der Halterung in den Innenschaft durch Einpressen oder Einschnappen kann ein Abdichtmittel in die Nut oder in den Kontaktbereich zwischen dem Innenschaft und der Halterung eingebracht werden, beispielsweise Silikon, was zu einer verbesserten Abdichtung führt.

Nachfolgend wird ein Ausführungsbeispiel anhand der beigefügten Figuren näher erläutert. Es zeigen:
Figur 1 - eine Halterung mit eingesetzter Elektrode in Draufsicht und Seitenansicht;
Figur 2 - eine perspektivische Darstellung der Elektrode in Draufsicht von oben und unten;
Figur 3 - eine Halterung ohne Elektrode von der Schaftinnenseite gesehen;
Figur 4 - die Rückseite der Halterung gemäß Figur 3;
Figur 5 - einen Innenschaft mit eingesetzter Elektrode; sowie
Figur 6 - einen teilgeschnittenen Außenschaft mit eingesetztem Innenschaft.

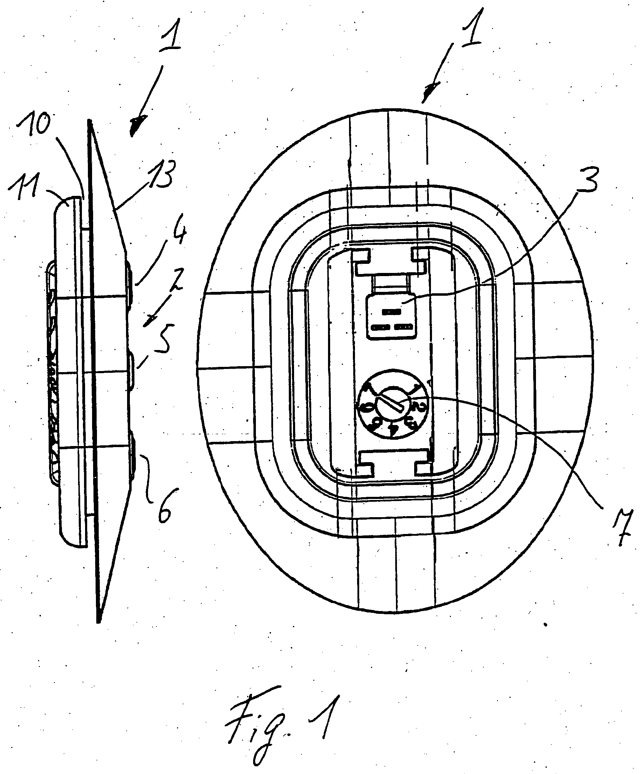

In der Figur 1 ist eine Halterung 1 mit einer darin eingesetzten Elektrode 2 gezeigt, die elektrischen Anschlüsse 3 zur Weiterleitung der über die Hautkontakte 4, 5 und 6 aufgenommenen Aktionspotentiale der Muskelgruppen. In der linken Seitenansicht ist der kalottenförmige Aufbau der zur Stumpfseite gerichteten Oberfläche 13 der Halterung 1, die ausgebildete Nut 10 und der Haltesteg 11 zu erkennen. In die Nut 10 wird ein Innenschaft eingesetzt. Durch das Eindrücken der Elektrodenhaltung von innen nach außen in eine Ausnehmung des Innenschaftes, die so groß ist, dass die Kontur innerhalb der Nut 10 aufgenommen und von der Innenseite der Halterung 1 und dem Haltesteg 11 überdeckt wird, wird eine luft- und wasserdichte Abdichtung der Ausnehmung innerhalb des Innenschaftes vorgenommen. Ein Drehschalter 7 auf der Rückseite der Elektrode ermöglicht eine Einstellung der montierten Elektrode 2 im angelegten Zustand.

Die Elektrode 2 kann in der Halterung eingespritzt, eingeklebt, eingepresst oder formschlüssig eingedrückt sein.

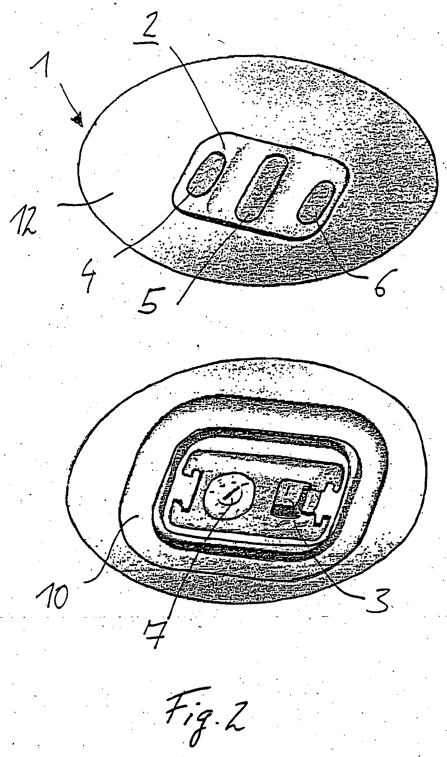

In der Figur 2 ist in einer perspektivischen Ansicht die fertig montierte Elektrode 2 in der Halterung 1 zu erkennen.

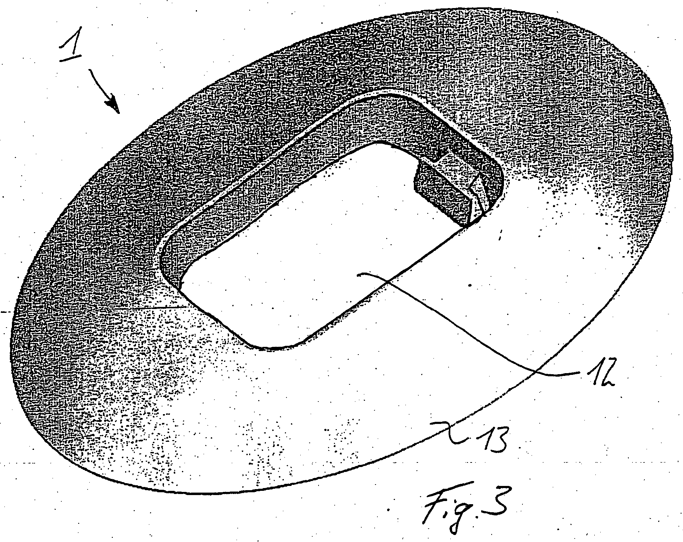

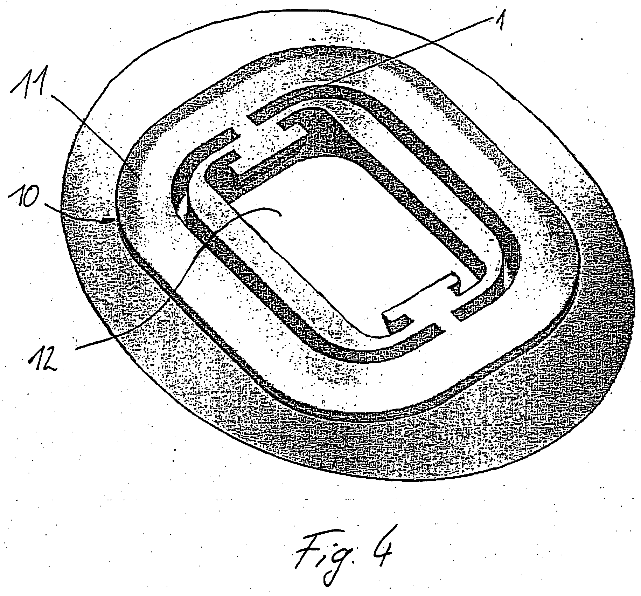

Bei einer mehrteiligen Ausgestaltung von Elektrode und Halterung wird die Halterung 1, wie es in den Figuren 3 oder 4 in unterschiedlichen Ansichten gezeigt ist, als Gussteil oder Spritzgussteil aus einem hautverträglichen Material, zum Beispiel Silikon gefertigt und mit einer Ausnehmung 12 zum Einsetzen der Ableitelektrode 2 versehen.

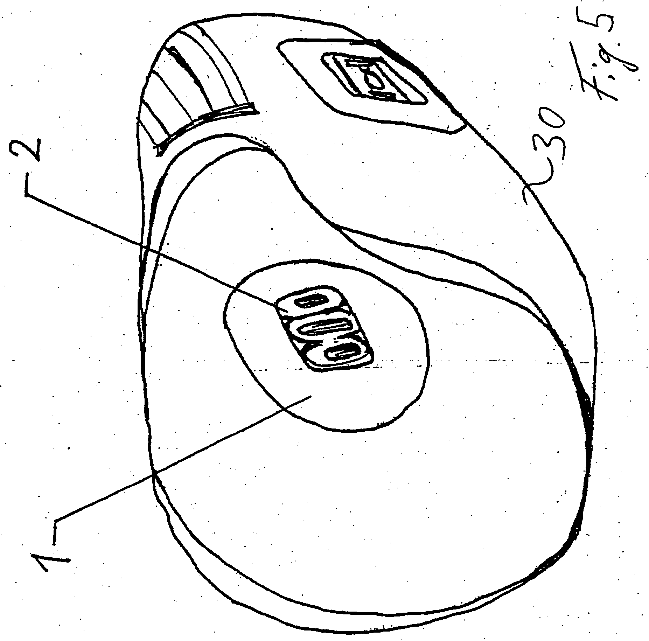

In der Figur 5 ist ein Innenschaft 30 von schräg hinten dargestellt, also aus der Einführrichtung des Stumpfes, vorliegend eines Armstumpfes. Der Innenschaft 30 ist als Saugschaft ausgebildet und weist an der Innenseite eine Elektrode 2 auf, die über eine kalottenförmige Halterung 1 an dem Innenschaft 30 festgelegt ist. Die Kontur der Halterung 1 auf der Innenseite des Innenschaftes 30 ist nur leicht erhaben ausgebildet, um einerseits ein möglichst angenehmes Traggefühl ohne Druckstellen zu ermöglichen und andererseits eine feste Anlage der Elektrode 2 an dem Stumpf zu gewährleisten, so dass auf Dauer elektromyografische Signale abgeleitet werden können. Wie oben beschrieben, ist die Halterung 1 mit einer umlaufenden Nut 10 versehen, in die der Innenschaft 30 eingreift, so dass die notwendige Ausnehmung durch Luft und Feuchtigkeit dicht verschlossen ist.

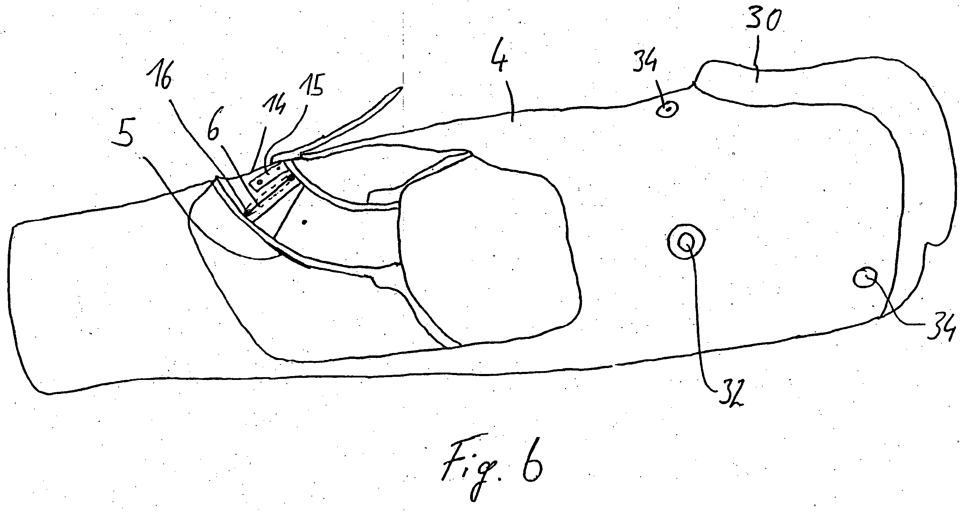

In der Figur 6 ist ein Prothesenteil mit einem Innenschaft 30 und einem Außenschaft 4 gezeigt, wobei der Außenschaft in einer Teilschnittdarstellung gezeigt ist. Der Innenschaft 30 ist über Schrauben 34 an dem stabilen Außenschaft 4 befestigt. Weiterhin ist eine Bohrung 32 in dem Außenschaft 4 vorgesehen, durch den die Elektrode 2, die im Innenschaft 30 festgelegt ist, an dem Drehschalter 7 eingestellt werden kann.

In der Teilschnittdarstellung ist zu erkennen, dass der Innenschaft 30 mit dem Außenschaft 4 über ein Verbindungsrohr 5 verbunden ist. Das Verbindungsrohr 5 ermöglicht ein Ausströmen der im Innenschaft 30 befindlichen Luft an einer geeigneten Stelle durch den Außenschaft 4 hindurch. Um ein Zurückströmen der Luft zu vermeiden, ist ein Einwegeventil 6 innerhalb des Verbindungsrohres 5 angeordnet. Durch Betätigen eines Hebels 15 legt sich ein O-Ring 16 an das Verbindungsrohr 5 an und dichtet somit den Innenschaft 3 luftdicht ab. Luft kann somit nur noch über das Einwegventil 6 ausströmen.

## Patentansprüche

1. Protheseninnenschaftsystem mit einem Innenschaft (3), der in einem Prothesenaußenschaft (4) mit Aufnahmeeinrichtungen für weitere Prothesenkomponenten verbindbar ausgebildet ist, **dadurch gekennzeichnet, dass** der Innenschaft (3) zumindest eine Ausnehmung zur Durchleitung myoelektrischer Signale aufweist, dass in der Ausnehmung eine dichtend abschließende Halterung (1) befestigt ist, dass in der Halterung (1) eine Elektrode (2) befestigt ist, dass an der Halterung (1) eine Nut (10) ausgebildet ist, in die der Innenschaft (3) eingreift und die Halterung (1) innenseitig und außenseitig den Rand der Ausnehmung des Innenschaftes (3) vollständig überdeckt.

2. Protheseninnenschaftsystem nach Anspruch 1, **dadurch gekennzeichnet, dass** die Halterung (1) aus einem elastischen Material, insbesondere Silikon, ausgebildet ist.

3. Protheseninnenschaftsystem nach Anspruch 1 oder 2, **dadurch gekennzeichnet, dass** die Elektrode (2) in der Halterung (1) eingespritzt, eingeklebt, eingepresst oder formschlüssig gehalten ist.

4. Protheseninnenschaftsystem nach einem der voranstehenden Ansprüche, **dadurch gekennzeichnet, dass** die dem Stumpf zugewandte Seite der Halterung (1) abgeflacht, insbesondere kalottenförmig ausgebildet ist.

5. Protheseninnenschaftsystem nach einem der voranstehenden Ansprüche, **dadurch gekennzeichnet, dass** an dem Innenschaft (3) ein Verbindungsrohr (5) zu dem Außenschaft (4) angeordnet ist.

6. Protheseninnenschaftsystem nach einem der voranstehenden Ansprüche, **dadurch gekennzeichnet, dass** an dem Innenschaft (3) ein Einwegeventil (6) befestigt ist, das im Innenschaft (3) befindliche Luft ausströmen lässt.

7. Protheseninnenschaftsystem nach Anspruch 5 und 6, **dadurch gekennzeichnet, dass** das Ventil (6) in dem Verbindungsrohr (5) angeordnet ist.

8. Protheseninnenschaftsystem nach einem der voranstehenden Ansprüche, **dadurch gekennzeichnet, dass** die Halterung (1) federnd ausgebildet ist.

## Claims

1. Prosthesis inner socket system, with an inner socket (3) which is designed to be connectable in a prosthesis outer socket (4) to receiving means for further prosthesis components, **characterized in that** the inner socket (3) has at least one recess for the passage of myoelectrical signals, **in that** a sealingly closing fitting (1) is secured in the recess, **in that** an electrode (2) is secured in the fitting (1), **in that** a groove (10) in which the inner socket (3) engages is formed on the fitting (1), and the fitting (1) completely covers the edge of the recess of the inner socket (3) on the inside and outside.

2. Prosthesis inner socket system according to Claim 1, **characterized in that** the fitting (1) is made from an elastic material, in particular silicone.

3. Prosthesis inner socket system according to Claim 1 or 2, **characterized in that** the electrode (2) is held in the fitting (1) by injection, adhesive bonding, pressure or by form-fit engagement.

4. Prosthesis inner socket system according to one of the preceding claims, **characterized in that** the side of the fitting (1) facing towards the stump is flattened, in particular in the shape of a spherical cap.

5. Prosthesis inner socket system according to one of the preceding claims, **characterized in that** a connecting tube (5) to the outer socket (4) is arranged on the inner socket (3).

6. Prosthesis inner socket system according to one of the preceding claims, **characterized in that** a one-way valve (6) is secured on the inner socket (3) and allows air located in the inner socket (3) to flow out.

7. Prosthesis inner socket system according to Claims 5 and 6, **characterized in that** the valve (6) is arranged in the connecting tube (5).

8. Prosthesis inner socket system according to one of the preceding claims, **characterized in that** the fitting (1) is designed to be resilient.

## Revendications

1. Système de prothèse à tige intérieure comprenant une tige intérieure (3) qui est réalisée de manière à être reliée dans une tige de prothèse extérieure (4) avec des moyens récepteurs pour d'autres composants de prothèse,
**caractérisé en ce que** la tige intérieure (3) comporte au moins un évidement pour le passage de signaux myoélectriques, **en ce qu'**une monture (1) assurant une fermeture étanche est fixée dans l'évidement,
**en ce qu'**une électrode (2) est fixée dans la monture (1), **en ce qu'**une gorge (10) est ménagée sur la monture (1), gorge dans laquelle s'engage la tige intérieure (3) et la monture (1) recouvre entièrement sur le côté intérieur et sur le côté extérieur la bordure de l'évidement de la tige intérieure (3).

2. Système de prothèse à tige intérieure selon la revendication 1, **caractérisé en ce que** la monture (1) est réalisée en un matériau élastique, en particulier en silicone.

3. Système de prothèse à tige intérieure selon la revendication 1 ou 2, **caractérisé en ce que** l'électrode (2) est intégrée par injection, par collage ou par pressage dans la monture (1) ou est maintenue dans celle-ci en coopération de formes.

4. Système de prothèse à tige intérieure selon l'une des revendications précédentes,
**caractérisé en ce que** le côté de la monture (1) tourné vers le moignon est réalisé aplati, en particulier en forme de calotte.

5. Système de prothèse à tige intérieure selon l'une des revendications précédentes,
**caractérisé en ce qu'**un tube de liaison (5) vers la tige extérieure (4) est agencé sur la tige intérieure (3).

6. Système de prothèse à tige intérieure selon l'une des revendications précédentes,
**caractérisé en ce qu'**un clapet antiretour (6) est fixé sur la tige intérieure (3), qui laisse s'échapper l'air qui se trouve dans la tige intérieure (3).

7. Système de prothèse à tige intérieure selon la revendication 5 et 6, **caractérisé en ce que** le clapet (6) est agencé dans le tube de liaison (5).

8. Système de prothèse à tige intérieure selon l'une des revendications précédentes, **caractérisé en ce que** la monture (1) est réalisée avec effet élastique.
